(19) **Europäisches Patentamt**

**European Patent Office**

**Office européen des brevets**

(11) **EP 1 294 395 B1**

(12) **EUROPEAN PATENT SPECIFICATION**

(45) Date of publication and mention
of the grant of the patent:
**19.04.2006 Bulletin 2006/16**

(51) Int Cl.:
***A61K 38/55*** *(2006.01)*      ***A61P 7/02*** *(2006.01)*

(21) Application number: **01938924.6**

(22) Date of filing: **06.06.2001**

(86) International application number:
**PCT/SE2001/001289**

(87) International publication number:
**WO 2001/095932 (20.12.2001 Gazette 2001/51)**

(54) **A COMBINATION PRODUCT COMPRISING MELAGATRAN AND A FACTOR VIIa INHIBITOR**

KOMBINATIONSPRODUKT ENTHALTEND MELAGATRAN UND EINEN FAKTOR-VIIIa INHIBITOR

PRODUIT COMBINE CONTENANT DU MELAGATRAN ET UN INHIBITEUR DU FACTEUR VII

(84) Designated Contracting States:
**AT BE CH CY DE DK ES FI FR GB GR IE IT LI LU MC NL PT SE TR**
Designated Extension States:
**SI**

(30) Priority: **10.06.2000 GB 0014134**

(43) Date of publication of application:
**26.03.2003 Bulletin 2003/13**

(73) Proprietor: **AstraZeneca AB**
**151 85 Södertälje (SE)**

(72) Inventors:
• **BYLUND, Ruth**
**S-431 83 Mölndal (SE)**
• **MATTSSON, Christer**
**S-431 83 Mölndal (SE)**

(56) References cited:
**WO-A1-97/39770**

• **MARGARETA ELG ET AL.: 'Effects of agents, used to treat bleeding disorders, on bleeding time prolonged by a very high dose of a direct thrombin inhibitor in anesthesized rats and rabbits' THROMBOSIS RESEARCH vol. 101, 2001, pages 159 - 170, XP002948831**

## Description

### Field of the Invention

This invention relates to a new combination of pharmaceutically-active compounds.

### Background and Prior Art

Blood coagulation is the key process involved in both haemostasis (i.e. the prevention of blood loss from a damaged vessel) and thrombosis (i.e. the formation of a blood clot in a blood vessel, sometimes leading to vessel obstruction).

Coagulation is the result of a complex series of enzymatic reactions.

One of the ultimate steps in this series of reactions is the conversion of the proenzyme prothrombin to the active enzyme thrombin.

Thrombin is known to play a central role in coagulation. It activates platelets, leading to platelet aggregation, converts fibrinogen into fibrin monomers, which polymerise spontaneously into fibrin polymers, and activates factor XIII, which in turn crosslinks the polymers to form insoluble fibrin. Furthermore, thrombin activates factor V and factor VIII leading to a "positive feedback" generation of thrombin from prothrombin.

International patent application WO 94/29336 discloses a group of thrombin-inhibiting compounds, including HOOC-CH$_2$-($R$)Cgl-Aze-Pab-H (in which Cgl represents cyclohexylglycine, Aze represents $S$-azetidine-2-carboxylic acid and Pab-H represents 4-aminomethylamidinobenzene), which is also known as melagatran (see Example 1 of WO 94/29336). International Patent Application WO 97/23499 discloses prodrugs of *inter alia* melagatran.

Factor VII is one (along with tissue factor) of the two proteins responsible for triggering the blood coagulation cascade *via* the extrinsic pathway. It is generally accepted that blood coagulation is physically initiated upon formation of a tissue factor/Factor VIIa complex. Once formed, this complex initiates coagulation by activating Factors IX and X.

Certain compounds are known to possess Factor VIIa inhibitory properties. See, for example, international patent applications WO 96/25427, WO 92/15686, WO 92/06711, WO 96/12021, WO 98/50420, WO 96/12800, WO 97/47651, WO 99/03498, WO 94/07528, WO 96/20689, WO 95/23860, WO 97/49684, WO 99/41276, WO 99/41231, WO 99/48878, WO 99/50254, WO 99/50257, WO 99/50263, WO 99/12936, WO 99/13063, WO 99/67215, WO 00/15658, WO 00/30646, WO 00/35858, WO 00/35886, WO 00/40601, WO 00/41531, WO 00/51989, WO 00/58346, WO 00/66545, WO 00/69826, WO 00/69832, WO 00/69834, WO 01/10892, WO 94/27631, WO 95/12674, WO 97/20939, WO 97/48687, WO 97/48706, WO 98/09987, WO 98/22619 and WO 99/00371; US patent Nos. 5,437,864, 5,833,982, 5,834,244, 5,863,893, 5,023,236, 6,020,331 and 5,639,739; European patent applications EP 921 116, EP 931 793, EP 669 317 and EP 987 274; and German Patent Applications Nos. DE 19829964, DE 19834751, DE 19835950, DE 19839499, DE 19845153, DE 19900355 and DE 19900471.

None of the above-mentioned documents disclose or suggest the administration of melagatran or a pharmaceutically-acceptable derivative thereof in conjunction with a Factor VIIa inhibitor. We have now found, surprisingly, that administration of just such a combination gives rise to a notable synergistic anticoagulant effect.

### Disclosure of the Invention

According to a first aspect of the invention there is provided a combination product comprising:

(A) melagatran or a salt, solvate, or prodrug thereof wherein the prodrug is of the formula

R$^1$O$_2$C-CH$_2$-($R$)Cgl-Aze-Pab-OH,

wherein R$^1$ represents C$_{1-10}$ alkyl or benzyl and the OH group replaces one of the amidino hydrogens in Pab; and
(B) a Factor VIIa inhibitor or a salt or solvate thereof,

wherein each of components (A) and (B) is formulated in admixture with a pharmaceutically-acceptable adjuvant, diluent or carrier.

The combination product according to the invention provides for the administration of melagatran (or salt, solvate or prodrug thereof) in conjunction with a Factor VIIa inhibitor (or salt or solvate thereof), and may thus be presented either as separate formulations, wherein at least one of those formulations comprises melagatran and at least one comprises Factor VIIa inhibitor, or may be presented (i.e. formulated) as a combined preparation (i.e. presented as a single formulation including melagatran and Factor VIIa inhibitor).

Thus, there is further provided:

(1) a pharmaceutical formulation including melagatran or a salt, solvate, or prodrug thereof wherein the prodrug is of the formula

$$R^1O_2C\text{-}CH_2\text{-}(R)Cgl\text{-}Aze\text{-}Pab\text{-}OH,$$

wherein $R^1$ represents $C_{1-10}$ alkyl or benzyl and the OH group replaces one of the amidino hydrogens in Pab, and a Factor VIIa inhibitor or a salt or solvate thereof, in admixture with a pharmaceutically-acceptable adjuvant, diluent or carrier (which formulation is hereinafter referred to as a "combined preparation"); and

(2) a kit of parts comprising components:

(a) a pharmaceutical formulation including melagatran or a salt, solvate, or prodrug thereof wherein the prodrug is of the formula

$$R^1O_2C\text{-}CH_2\text{-}(R)Cgl\text{-}Aze\text{-}Pab\text{-}OH,$$

wherein $R^1$ represents $C_{1-10}$ alkyl or benzyl and the OH group replaces one of the amidino hydrogens in Pab, in admixture with a pharmaceutically-acceptable adjuvant, diluent or carrier; and
(b) a pharmaceutical formulation including a Factor VIIa inhibitor or a salt or solvate thereof, in admixture with a pharmaceutically-acceptable adjuvant, diluent or carrier,

which components (a) and (b) are each provided in a form that is suitable for administration in conjunction with the other.
According to a further aspect of the invention, there is provided a method of making a kit of parts as defined above, which method comprises bringing a component (a), as defined above, into association with a component (b), as defined above, thus rendering the two components suitable for administration in conjunction with each other.
By bringing the two components "into association with" each other, we include that components (a) and (b) of the kit of parts may be:

(i) provided as separate formulations (i.e. independently of one another), which are subsequently brought together for use in conjunction with each other in combination therapy; or
(ii) packaged and presented together as separate components of a "combination pack" for use in conjunction with each other in combination therapy.

Thus, there is further provided a kit of parts comprising:

(I) one of components (a) and (b) as defined herein; together with
(II) instructions to use that component in conjunction with the other of the two components.

The kits of parts described herein may comprise more than one formulation including an appropriate quantity/dose of melagatran, salt, solvate or prodrug thereof, and/or more than one formulation including an appropriate quantity/dose of Factor VIIa inhibitor, salt or solvate thereof, in order to provide for repeat dosing. If more than one formulation. (comprising either active compound) is present, such formulations may be the same, or may be different in terms of the dose of melagatran (or salt, solvate or prodrug) or Factor VIIa inhibitor (or salt or solvate), chemical composition and/or physical form.
A further aspect of the invention provides the use of a pharmaceutical formulation including melagatran (or a salt, solvate, or prodrug thereof wherein the prodrug is of the formula

$$R^1O_2C\text{-}CH_2\text{-}(R)Cgl\text{-}Aze\text{-}Pab\text{-}OH,$$

wherein $R^1$ represents $C_{1-10}$ alkyl or benzyl and the OH group replaces one of the amidino hydrogens in Pab), and a Factor VIIa inhibitor (or a salt or solvate thereof), in admixture with a pharmaceutically-acceptable adjuvant, diluent or carrier for the manufacture of a medicament for a method of treatment of a condition where anticoagulant therapy is indicated.
A further aspect of the invention provides the use of

(a) a pharmaceutical formulation including melagatran or a salt, solvate, or prodrug thereof wherein the prodrug is of the formula

R$^1$O$_2$C-CH$_2$-(R)Cgl-Aze-Pab-OH,

wherein R$^1$ represents C$_{1-10}$ alkyl or benzyl and the OH group replaces one of the amidino hydrogens in Pab, in admixture with a pharmaceutically-acceptable adjuvant, diluent or carrier; in conjunction with

(b) a pharmaceutical formulation including a Factor VIIa inhibitor or a salt or solvate thereof, in admixture with a pharmaceutically-acceptable adjuvant, diluent or carrier,

for the manufacture of medicament for a method of treatment of a condition where anticoagulant therapy is indicated (by which we mean where anticoagulation is required).

For the avoidance of doubt, as used herein, the term "treatment" includes therapeutic and/or prophylactic treatment.

With respect to the kits of parts as described herein, by "administration in conjunction with", we include that respective formulations comprising melagatran (or salt, solvate or prodrug thereof) and Factor VIIa inhibitor (or) salt or solvate thereof) are administered, sequentially, separately and/or simultaneously, over the course of treatment of the relevant condition, which condition may be acute or chronic.

Thus, in respect of the combination product according to the invention, the term "administration in conjunction with" includes that the two components of the combination product (melagatran/salt solvate or prodrug and Factor VIIa inhibitor/salt or solvate) are administered (optionally repeatedly), either (in the case of a combined preparation) together, or (in the case of a kit of parts) sufficiently closely in time, to enable a beneficial effect for the patient, that is greater, over the course of the treatment of the relevant condition, than if either a formulation comprising melagatran/salt, solvate or prodrug or a formulation comprising Factor VIIa inhibitor/salt or solvate, are administered (optionally repeatedly) alone, in the absence of the other component, over the same course of treatment. Determination of whether a combination provides a greater beneficial effect in respect of, and over the course of treatment of, a particular condition, will depend upon the condition to be treated or prevented, but may be achieved routinely by the skilled person.

Further, in the context of a kit of parts according to the invention, the term "in conjunction with" includes that one or other of the two formulations may be administered (optionally repeatedly) prior to, after, and/or at the same time as, administration with the other component. When used in this context, the terms "administered simultaneously" and "administered at the same time as" include that individual doses of melagatran (or salt, solvate or prodrug thereof) and Factor VIIa inhibitor (or salt or solvate thereof) are administered within 48 hours (e.g. 24 hours) of each other.

"Pharmaceutically-acceptable salts and solvates" of melagatran and Factor VIIa inhibitors includes pharmaceutically-acceptable non-toxic organic or inorganic acid addition salts. It will be appreciated that the term further includes derivatives that have the same biological function and/or activity as melagatran, or the Factor VIIa inhibitor, as appropriate. "Prodrugs" of melagatran include any composition of matter that, following oral or parenteral administration, is metabolised *in vivo* to form melagatran, in an experimentally-detectable amount, and within a predetermined time (e.g. within a dosing interval of between 6 and 24 hours (i.e. once to four times daily)). For the avoidance of doubt, the term "parenteral" adminstration includes all forms of adminstration other than oral administration.

Factor VIIa inhibitors that may be employed in the combination products according to the invention include those described international patent applications WO 96/25427, WO 92/15696, WO 92/06711, WO 96/12021, WO 98/50420, WO 96/12800, WO 97/47651, WO 99/03498, WO 94/07528, WO 96/20689, WO 95/23860, WO 97/49684, WO 99/41276, WO 99/41231, WO 99/48878, WO 99/50254, WO 99/50257, WO 99/50263, WO 99/12936, WO 99/13063, WO 99/67215, WO 00/15658, WO 00/30646, WO 00/35858, WO 00/35886, WO 00/40601, WO 00/41531, WO 00/51989, WO 00/58346, WO 00/66545, WO 00/69826, WO 00/69832, WO 00/69834, WO 01/10892, WO 94/27631, WO 95/12674, WO 97/20939, WO 97/48687, WO 97/48706, WO 98/09987, WO 98/22619 and WO 99/00371; US patent Nos. 5,437,864, 5,833,982, 5,834,244, 5,863,893, 5,023,236, 6,020,331 and 5,639,739; European patent applications EP 921 116, EP 931 793, EP 669 317 and EP 987 274; and German Patent Applications Nos. DE 19829964, DE 19834751, DE 19835950, DE 19839499, DE 19845153, DE 19900355 and DE 19900471, the specific and generic disclosures in all of which documents are hereby incorporated by reference.

Preferred Factor VIIa inhibitors include those described in international patent applications WO 98/50420, WO 97/47651, WO 99/03498, WO 96/20689, WO 00/15658, WO 00/35858, WO 00/35886, WO 00/41531, WO 00/58346, WO 00/66545, WO 00/69826, WO 00/69832, WO 00/69834, WO 98/22619 and US patent No. 5,639,739, and especially those described in international patent applications WO 92/15686, WO 96/12021 and WO 99/41231 and European patent applications EP 987 274 and EP 921 116, the specific and generic disclosures in all of which documents are hereby incorporated by reference, as well as *N*-[(3-carboxybenzyl)sulfonyl]-D-valyl-*N*$^1$-{4-[amino(imino)methyl]benzyl}-L-leucinamide and pharmaceutically-acceptable derivatives thereof, which may be prepared according to the method described below.

The term "condition where anticoagulant therapy is indicated" will be understood by those skilled in the art to include the following:

The treatment and/or prophylaxis of thrombosis and hypercoagulability in blood and/or tissues of animals including man. It is known that hypercoagulability may lead to thrombo-embolic diseases. Conditions associated with hypercoagulability and thrombo-embolic diseases which may be mentioned include inherited or acquired activated protein C resistance, such as the factor V-mutation (factor V Leiden), and inherited or acquired deficiencies in antithrombin III, protein C, protein S, heparin cofactor II. Other conditions known to be associated with hypercoagulability and thrombo-embolic disease include circulating antiphospholipid antibodies (Lupus anticoagulant), homocysteinemi, heparin induced thrombocytopenia and defects in fibrinolysis, as well as coagulation syndromes (e.g. disseminated intravascular coagulation (DIC)) and vascular injury in general (e.g. due to surgery).

The treatment of conditions where there is an undesirable excess of thrombin without signs of hypercoagulability, for example in neurodegenerative diseases such as Alzheimer's disease.

Particular disease states which may be mentioned include the therapeutic and/or prophylactic treatment of venous thrombosis (e.g. DVT) and pulmonary embolism, arterial thrombosis (e.g. in myocardial infarction, unstable angina, thrombosis-based stroke and peripheral arterial thrombosis), and systemic embolism usually from the atrium during atrial fibrillation or from the left ventricle after transmural myocardial infarction, or caused by congestive heart failure; prophylaxis of re-occlusion (ie thrombosis) after thrombolysis, percutaneous trans-luminal angioplasty (PTA) and coronary bypass operations; the prevention of re-thrombosis after microsurgery and vascular surgery in general.

Further indications include the therapeutic and/or prophylactic treatment of disseminated intravascular coagulation caused by bacteria, multiple trauma, intoxication or any other mechanism; anticoagulant treatment when blood is in contact with foreign surfaces in the body such as vascular grafts, vascular stents, vascular catheters, mechanical and biological prosthetic valves or any other medical device; and anticoagulant treatment when blood is in contact with medical devices outside the body such as during cardiovascular surgery using a heart-lung machine or in haemodialysis; the therapeutic and/or prophylactic treatment of idiopathic and adult respiratory distress syndrome, pulmonary fibrosis following treatment with radiation or chemotherapy, septic shock, septicemia, inflammatory responses, which include, but are not limited to, edema, acute or chronic atherosclerosis such as coronary arterial disease and the formation of atherosclerotic plaques, cerebral arterial disease, cerebral infarction, cerebral thrombosis, cerebral embolism, peripheral arterial disease, ischaemia, angina (including unstable angina), reperfusion damage, restenosis after percutaneous trans-luminal angioplasty (PTA) and coronary artery bypass surgery.

Preferred conditions include thrombosis.

In accordance with the invention, melagatran, prodrugs thereof, Factor VIIa inhibitors and salts or solvates of either, may be administered orally, intravenously, subcutaneously, buccally, rectally, dermally, nasally, tracheally, bronchially, topically, by any other parenteral route, or via inhalation, in the form of a pharmaceutical preparation comprising melagatran and/on Factor VIIa inhibitor in a pharmaceutically-acceptable dosage form. Depending on the disorder, and the patient, to be treated, as well as the route of administration, the compositions may be administered at varying doses.

Preferred modes of delivery are systemic. For melagatran and salts or solvates thereof, preferred modes of administration are parenteral, more preferably intravenous, and especially subcutaneous. For prodrugs of melagatran, preferred modes of administration are oral.

In the therapeutic treatment of mammals, and especially humans, melagatran, Factor VIIa inhibitors, and derivatives of either will generally be administered as a pharmaceutical formulation in admixture with a pharmaceutically-acceptable adjuvant, diluent or carrier, which may be selected with due regard to the intended route of administration and standard pharmaceutical practice.

Suitable formulations for use in administering melagatran and salts, solvates or prodrugs thereof are described in the literature, for example as described in *inter alia* international patent applications WO 94/29336, WO 96/14084, WO 96/16671, WO 97/23499, WO 97/39770, WO 97/45138, WO 98/16252, WO 99/27912, WO 99/27913, WO 00/12043 and WO 00/13671, the disclosures in which documents are hereby incorporated by reference.

Similarly, suitable formulations for use in administering Factor VIIa inhibitors and salts or solvates thereof are described in the literature, for example as described in the prior art documents relating to Factor VIIa inhibitors that are mentioned hereinbefore, the disclosures in which documents are hereby incorporated by reference. Otherwise, the preparation of suitable formulations, and in particular combined preparations including both melagatran/salt, solvate or prodrug and Factor VIIa inhibitor/salt or solvate may be achieved non-inventively by the skilled person using routine techniques.

The amounts of melagatran, prodrug thereof, Factor VIIa inhibitor, or salt or solvate of either, in the respective formulation(s) will depend on the severity of the condition, and on the patient, to be treated, as well as the compound(s) which is/are employed, but may be determined non-inventively by the skilled person.

Suitable doses of melagatran, prodrug thereof, Factor VIIa inhibitors and salt or solvate of either, in the therapeutic and/or prophylactic treatment of mammalian, especially human, patients may be determined routinely by the medical practitioner or other skilled person, and include the respective doses discussed in the prior art documents relating to melagatran (or salts, solvates or prodrugs) thereof and to Factor VIIa inhibitors, that are mentioned hereinbefore, the disclosures in which documents are hereby incorporated by reference.

In the case of melagatran, suitable doses of active compound, prodrugs and salts or solvates thereof, in the therapeutic and/or prophylactic treatment of mammalian, especially human, patients include those which give a mean plasma concentration of up to 5 μmol/L, for example in the range 0.001 to 5 μmol/L over the course of treatment of the relevant condition. Suitable doses may thus be in the range 0.1 mg once daily to 25 mg three times daily, and/or-up to 100 mg infused parenterally over a 24 hour period, for melagatran, and in the range 0.1 mg once daily to 100 mg three times daily for prodrugs of melagatran including those specifically mentioned herein.

In the case of Factor VIIa inhibitors, suitable doses for therapeutic or prophylactic purposes are in the range such that, for example, 10 to 500 mg is received, given if required in divided doses. In general lower doses will be administered when a parenteral route is employed, for example a dose for intravenous administration in the range, for example, 1 to 50 mg will generally be used. At continuous infusion, a dose of between 0.1 and 5 mg/kg/hour will generally be used.

In any event, the physician, or the skilled person, will be able to determine the actual dosage which will be most suitable for an individual patient, which is likely to vary with the condition that is to be treated, as well as the age, weight, sex and response of the particular patient to be treated. The above-mentioned dosages are exemplary of the average case; there can, of course, be individual instances where higher or lower dosage ranges are merited, and such are within the scope of this invention.

When separate formulations are administered, the sequence in which the formulations comprising melagatran (or salt, solvate or prodrug thereof), and Factor VIIa inhibitor (or salt or solvate thereof), may be administered (i.e. whether, and at what point, sequential, separate and/or simultaneous administration takes place) may be determined by the physician or skilled person. For example, the sequence may depend upon many factors that will be evident to the skilled person, such as whether, at any time during the course or period of treatment, one or other of the formulations cannot be administered to the patient for practical reasons (e.g. the patient is unconscious and thus unable to take an oral formulation comprising either melagatran or Factor VIIa inhibitor).

The methods described herein may have the advantage that, in the treatment of conditions where anticoagulant therapy is indicated, they may be more convenient for the physician and/or patient than, be more efficacious than, be less toxic than, have a broader range of activity than, be more potent than, produce fewer side effects than, or that they may have other useful pharmacological properties over, similar methods known in the prior art for the treatment of such conditions.

The invention is illustrated, but in no way limited, by the following examples.

Example 1

N-[(3-Carboxybenzyl)sulfonyl]-D-valyl-$N^1$- {4-[amino (imino) methyl]-benzyl}-L- leucinamide

(a) $N^2$-(*tert*-Butoxycarbonyl)-$N^1$-(4-cyanobenzyl)-L-leucinamide

To a stirred solution on an ice bath of 4-aminomethylbenzonitrile (15.0 g, 41.0 mmol), DMAP (37.0 g 303 mmol) and EDC (13.8 g, 72 mmol) in DMF (200 mL) was added 2-*tert*-(L)-butoxycarbonylamino-4-methyl-pentanoic acid (5.4 g). The ice bath was removed. Water (500 mL) was added after 24 hours. The product was extracted with DCM (500 mL). The organic phase was washed with 1 M HCl (2 x 250 mL), $H_2O$ (250 mL), 1 M $NaHCO_3$ (2 x 250 mL), $H_2O$ (250 mL) and brine (250 mL). The organic layer was dried over $MgSO_4$, filtered and concentrated to give 16.92 g of dry solid. Yield 75 %.
$^1$H-NMR (400 MHz, DMF-$d_7$) δ 0.71 (d, 3H), 0.74 (d, 3H), 1.22 (s, 9H), 1.41 (m, 2H), 1.69 (m, 1H), 3.98 (m, 1H), 4.31 (d, 2H), 6.73 (d, 1H), 7.36 (d, 2H), 7.61 (d, 2H), 8.35 (t, 1H)
MS m/e 346 (M+H)$^+$

(b) $N^1$-(4-Cyanobenzyl)-L-leucinamide

To a reaction flask on an ice bath containing $N^2$-(*tert* butoxycarbonyl)-$N^1$-(4-cyanobenzyl)-L-leucinamide (16.9 g, 48.9 mmol, see step (a) above) was added dropwise HCl-saturated EtOAc (250 mL). The ice bath was removed. After 30 min., the reaction was evaporated to give the HCl-salt of the sub-title product (13.0 g, 46.1 mmol), Yield 94 %.
$^1$H-NMR (400 MHz, DMF-$d_7$) δ 0.77 (d, 3H), 0.79 (d, 3H), 1.66 (m, 3H), 3.99 (m, 1H), 4.35 (ddd, 2H), 7.45 (d, 2H), 7.63 (d, 2H), 8.77 (broad, s, 3H)
MS m/e 246 (M+H)$^+$

(c) N-{[3-(Methoxycarbonyl)benzyl]sulfonyl}-D-valyl-$N^1$-(4-cyanobenzyl)-L- leucinamide

To a stirred solution, cooled on ice bath, of $N^1$-(4-cyanobenzyl)-L-leucinamide (0.56 g, 2.0 mmol, see step (b) above), DMAP (1.1 g, 9 mmol) and EDC (0.42 g, 2.2 mmol) in DMF (10 mL) was added N-{[3-(methoxycarbonyl)benzyl]sulfonyl}-D-valine (0.60 g, 1.8 mmol). The ice bath was removed and, after 24 hours, $H_2O$ (80 mL) was added. The mixture was extracted with EtOAc (3 x 30 mL). The combined organic phase was washed with 1M HCl (2 x 100 mL), $H_2O$ (100 mL), 1M $NaHCO_3$ (2 x 100 mL), $H_2O$ (100 mL) and brine (100 mL). The organic phase was separated and dried over $MgSO_4$, filtered and concentrated *in vacuo* to give 0.79 g of sub-title compound as a dry solid. Yield 71%.

$^1$H-NMR (400 MHz, $CDCl_3$) δ 0.92 (d, 3H), 0.94 (d, 3H), 0.95 (d, 3H), 0.98 (d, 3H), 1.60 (m, 2H), 1.80 (m, 1H), 1.95 (m, 1H), 3.44 (m, 1H), 3.93 (s, 3H), 4.23 (dd, 2H), 4.44 (ddd, 2H), 4.60 (m, 1H), 6.29 (d, 1H), 7.33 (d, 2H), 7.50 (m, 3H), 8.04 (d, 2H)

MS m/e 557 (M+H)[+]

(d) <u>N-[(3-Carboxybenzyl)sulfonyl]-D-valyl-N[1]-(4-cyanobenzyl)-L-leucinamide</u>

To a stirred solution of N-{[3-(methoxycarbonyl)benzyl]sulfonyl}-D-valyl-N[1]-(4-cyanobenzyl)-L-leucinamide (0.76 g, 1.37 mmol, see step (c) above) in THF was added dropwise a 0.4M lithium hydroxide solution in $H_2O$ (20 mL). After 2 h, the pH of the reaction was adjusted to 7 with 1M HCl. The reaction mixture was evaporated to dryness *in vacuo* (1.06 g). The crude product, contaminated with LiCl, was used directly in the next step without further purification.

$^1$H-NMR (400 MHz, MeOD) δ 0.90 (d, 3H), 0.91 (d, 3H), 0.95 (d, 3H), 0.96 (d, 3H), 1.65 (m, 3H), 1.91 (m, 1H), 3.52 (d, 1H), 3.72 (m, 1H), 4.35 (m, 4H), 7.39 (m, 4H), 7.58 (d, 2H), 7.92 (d, 1H), 7.97 (s, 1H)

MS m/e 543 (M+H)[+]

(e) <u>N-[(3-Carboxybenzyl)sulfonyl]-D-valyl-N[1]- {4-[(Z)-amino (hydroxyimino) methyl] benzyl}-L- leucinamide</u>

To a stirred solution of *N*-[(3-carboxybenzyl)sulfonyl]-D-valyl-*N*[1]-(4-cyanobenzyl)-L-leucinamide (0.89 g, crude, see step (d) above) in ethanol under nitrogen atmosphere was added triethylamine (0.74 mL) and hydroxylamine hydrochloride (0.22 g). The reaction mixture was heated to reflux. After 18 hours, the reaction mixture was concentrated to dryness under vacuum (0.74 g). The crude product was used directly in the next step without further purification.

MS m/e 576 (M+H)[+]

(f) <u>N-[(3-Carboxybenzyl)sulfonyl]-D-valyl-N[1]- {4-[amino (imino) methyl]-benzyl}-L- leucinamide</u>

To a stirred solution of *N*-[(3-carboxybenzyl)sulfonyl]-D-valyl-*N*[1]-{4-[(Z)-amino(hydroxyimino)methyl]benzyl}-L-leucinamide (0.74 g, crude, see step (e) above) in acetic acid (40 mL) was added 10 % Pd/C (0.13 g) and acetic anhydride (0.13 mL). The reaction was carried out under a hydrogen atmosphere (5 bar). After 3.5 hours, the reaction mixture was filtered through celite. The celite was washed with acetic acid, the combined filtrate was concentrated *in vacuo* and the residue was precipitated from acetonitrile/1M $NH_4OAc$, filtered and dried to give 0.34 g of title product.

$^1$H-NMR (400 MHz, $CD_3COOD$) rotamers δ 0.91 (d, 3H), 0.95 (d, 3H), 0.96 (d, 3H), 0.98 (d, 3H), 1.71 (m, 2H), 1.96 (m, 1H), 3.71 (d, 1H, major rotamer), 3.74 (d, 1H, minor rotamer), 4.26 (d, 2H, minor rotamer), 4.32 (d, 2H, major rotamer), 4.45 (d, 1H), 4.60 (d, 1H), 4.70 (m, 1H), 7.46 (m, 3H), 7.66 (d, 1H), 7.73 (d, 2H, major rotamer), 7.77 (d, 2H, minor rotamer), 8.05 (broad s, 2H)

MS m/e 560 (M+H)[+]

**Abbreviations**

DCM = dichloromethane
DMF = dimethylformamide
DMAP = 4-dimethylaminopyridine
EDC = 1-(3-dimethylaminopropyl)-3-ethylcarbodiimide hydrochloride
MS = mass spectrometry

The compound of Example 1 was Tested in the following Tests.

<u>Test A</u>

<u>Chromogenic Robot Method for Factor VIIa:</u>

The chromogenic substrate Spectrozyme FVIIa, $CH_3SO_2$-D-CHA-But-Arg-pNA (pefachrome 5979 from Pentapharm, Switzerland) was purchased from American Diagnostica Inc. (Greenwich, CT, USA). The $K_M$ under the conditions of the assay was determined separately. Recombinant human factor VIIa (rFVIIa, product no 407) and non-lipidated recombinant human tissue-factor (rTF, product no 4500) were purchased from American Diagnostica Inc. The stock solutions of rFVIIa (1.08 mg protein dissolved with 1 mL water to 21.6 $\mu$M in 20 mM Tris-HCl, 100 mM NaCl, pH 7.4) and rTF (1 mg protein with 1.5 mL water to 19 $\mu$M in 50 mM Tris-HCl, pH 8.0, 150 mM NaCl, CHAPS (3-((3-cholamidopropyl)dimethylammoniol)-1-propanesulphonate; 10 mM) and 200 mM mannitol) were stored in 0.5 mL reagent tubes at -80°C. A mixture was made of 29 nM rFVIIa with 68 nM TF by addition of 8 $\mu$L of 21.6 $\mu$M rFVIIa and 22 $\mu$L of 19 $\mu$M rTF to 6 mL assay buffer with 0.1% bovine serum albumin (BSA). The mixture was incubated at room temperature for 30 min. The assay buffer contained 50 mM Tris with 100 mM NaCl, 0.1% BSA, 5 mM CaCl$_2$ adjusted to pH 7.4 with HCl at 37°C. The compound was dissolved in 100% DMSO to a concentration of 10 mM in a 96-well microtiter plate. A new plate was prepared by dilution in steps of 1:3 with DMSO to finally 10 concentrations in column 2 to column 11. In the final assay, 4 $\mu$L was transferred from all the wells of the plate to the measuring plate to the final assay concentrations with 2.7% DMSO in 150 $\mu$L/well. The factor VIIa robot assay was performed in a ROSYS Plato 3000 Robotic microtiter plate processor in a total assay volume of 150 $\mu$L. To the 4 $\mu$L inhibitor in column 2-11 was added 122 $\mu$L of assay buffer at room temperature, then 12 $\mu$L of the FVIIa-rTF working solution was multi-dispensed to all wells, and finally after mixing 12 $\mu$L of the Spectrozyme FVIIa working solution to a final concentration of 0.2 mM. After mixing and incubation for 40 minutes at 37°C, the optical absorbance difference was read at 405 nm. The IC$_{50}$ value for each compound was calculated by non-linear regression to the equation:

$$A_I/A_0 = 1/(1+([I]/IC_{50})^s)$$

Where $A_I$ = the final absorbance at 405 nm in the presence of the inhibitor, $A_0$ = in the absence of inhibitor, and s the slope in the dose titration curve, which has the theoretical value of 1 for a pure competitive inhibitor. The $K_i$ values can then be estimated from the relationship:

$$K_i = IC_{50}/(1+ S/K_m) = IC_{50}/1.32$$

Test B

**Determination of Thrombin Inhibition with a Chromogenic, Robotic Assay**

Thrombin inhibitor potency was measured with a chromogenic substrate method, in a Plato 3300 robotic microplate processor (Rosys AG, CH-8634 Hombrechtikon, Switzerland), using 96-well, half volume microtitre plates (Costar, Cambridge, MA, USA; Cat No 3690). Stock solutions of test substance in DMSO (72 $\mu$L), 0.1 - 1 mmol/L, were diluted serially 1:3 (24 + 48 $\mu$L) with DMSO to obtain ten different concentrations, which were analysed as samples in the assay. 2 $\mu$L of test sample was diluted with 124 $\mu$L assay buffer, 12 $\mu$L of chromogenic substrate solution (S-2366, Chromogenix, Mölndal, Sweden) in assay buffer and finally 12 $\mu$L of $\alpha$-thrombin solution (Human $\alpha$-thrombin, Sigma Chemical Co. or Hematologic Technologies) in assay buffer, were added, and the samples mixed. The final assay concentrations were: test substance 0.00068 - 13.3 $\mu$mol/L, S-2366 0.30 mmol/L, $\alpha$-thrombin 0.020 NIHU/mL. The linear absorbance increment during 40 minutes incubation at 37°C was used for calculation of percentage inhibition for the test samples, as compared to blanks without inhibitor. The IC$_{50}$ robotic value, corresponding to the inhibitor concentration which causes 50 % inhibition of the thrombin activity, was calculated from a log concentration vs. % inhibition curve.

Test C

Prothrombin Time (PT) assay

The following reagents were prepared: An aliquot of a frozen human plasma pool, containing citrate as an anticoagulant, was rapidly thawed at 37°C. A 10 mM stock solution of the inhibitor in DMSO was diluted with saline containing 0.1% BSA to a final 1% of DMSO. Series dilutions were made in steps of 1:3 for a dose response titration. Thromborel® S from Dade Behring GmbH (Marburg, Germany), was reconstituted 1:10 with 25 mM CaCl$_2$, according to the instructions of the manufacturer. 2.5 $\mu$L of inhibitor was mixed with 22.5 $\mu$L human plasma, incubated for 1 min. at 37°C in the cup of an Amelung KC10 microcoagulometer from Heinrich Amelung GmbH (Lemgo, Germany). Coagulation was initiated by addition of 50 $\mu$L Thromborel S, which was pre-warmed at 37°C. The time to clot was determined (CT$_I$) for each inhibitor concentration ([I]) *in duplo.* A titration curve was made by plotting the inverse clotting time versus the inhibitor concentration. The $IC_{50}$ value, the time to double the clotting time, was determined by non-linear regression to the equation:

$$CT_0/CT_I = 1/(1 + ([I]/IC_{50})^s)$$

where "$CT_0$" = the clotting time without inhibitor added, and "s" "the slope.

The results for the title compound of Example 1 are:

Test A - $IC_{50}$ (Factor VIIa) = 64 nM
Test B - $IC_{50}$ (thrombin) = 6.2 $\mu$M
Test C - $IC_{50}$ (PT) = 3.8 $\mu$M

indicating that the compound in question is a selective inhibitor of Factor VIIa.

Example 2

Synergistic Effect of Melagatran and a Factor VIIa Inhibitor

Human blood from healthy volunteers, both men and women, was collected in 50 mL conical plastic tubes containing 5 mL of 0.13 mol/L of buffered tri-sodium citrate. The blood was immediately chilled on ice and centrifuged at 200 x g for 20 minutes. Platelet poor plasma was then divided into fractions of 10 mL each and "spiked" with melagatran (Compound A) or with the title compound of Example 1 above (Compound B) to a final concentration that prolonged APTT by approximately 20 % , 40 % , 60 % and 100%, respectively.

APTT was determined as follows: 100 $\mu$L of pre-warmed APTT reagent (Stago Diagnostica) was added to 100 $\mu$L of pre-warmed plasma and incubated for 180 s at 37°C. Coagulation was initiated by the addition of 100 $\mu$L of $CaCl_2$ (0.025 mol/L). The clotting time was measured with a KC 10 Coagulometer, coupled to a CR-A calculator, CR 10 printer and PR60 heat block (Amelung GmbH, Lemago Germany). Each clotting test was performed in duplicate and the mean clotting time was used.

APTT for Compound A and Compound B were tested at 4 different concentrations and the results are shown in Table 1, in which Cp A and Cp B are the plasma concentrations of Compounds A and B, respectively. APTT values are expressed in seconds and, within brackets, as percentage prolongation over the baseline value.

Table 1

| Cp A ($\mu$mol/L) | APTT (s) | Cp B ($\mu$mol/L) | APTT (s) |
| --- | --- | --- | --- |
| 0.00 | 36.8 | 0.00 | 35.7 |
| 0.08 | 45.8 (24%) | 1.50 | 45.2(26%) |
| 0.18 | 53.8 (46%) | 3.20 | 51.5 (44%) |
| 0.27 | 59.2 (61%) | 4.90 | 57.7 (62%) |
| 0.56 | 72.1 (96%) | 8.30 | 73.3(105%) |

APTT correlated well to a linear function within the tested concentration intervals for both compounds, and can be described by the following equations:

$$\text{Compound A: } y = 52.9x + 43.3 \ R^2 = 0.98 \ldots\ldots (\text{Eq 1})$$

$$\text{Compound B: } y = 4.2x + 38.2 \quad R^2 = 1.00 \ldots\ldots (\text{Eq 2})$$

In order to test a possible synergistic effect between Compounds A and B the following model was used. The concentrations of Compounds A and B that caused about 20 %, 40 % and 60 % prolongation of APTT were first determined (Table 1). Equipotent concentrations of Compounds A and B, e.g. 0.08 $\mu$mol/L of A and 1.5 $\mu$mol/L of B, were then mixed and APTT for the mixtures were determined as above. An indication of synergy is obtained if APTT for this mixture is longer than APTT for a "mixture" of equipotent concentrations of Compound A itself (i.e. Compound A + Compound A at a concentration that prolongs APTT by 20% (0.08 $\mu$mol/L + 0.08 $\mu$mol/L = 0.16 $\mu$mol/L)). However, a true synergistic effect between two compounds can never be established unless the dose response curves for both individual compounds are known and all data applied to the algebraic model of Berenbaum's equation (see *Clin. Exp. Immunol.* (1977) **28,** 18):

$$(\text{Cp A in mixture/Ae}) + (\text{Cp B in mixture/Be}) < 1$$

In this equation, CpA and CpB are the plasma concentrations of Compounds A and B, respectively in the mixture, and Ae and Be are individual concentrations of Compounds A and B that are required to obtain the same APTT results as that of the mixture. These concentrations can be calculated from the linear dose-responses for Compounds A and B, respectively (Equations 1 and 2). All APTT results from experiments with Compound A alone or in combination with Compound B are summarised in Table 2.

Table 2

| Compound A + A $\mu$mol/L | APTT sec. | Compound A + B $\mu$mol/L | APTT sec. | A | B |
|---|---|---|---|---|---|
| | | | | $\mu$mol/L | |
| 0.08 + 0.08 = 0.16 | 51.8 | 0.08 (A) + 1.5 (B) | 54.3 | 0.21 | 3.87 |
| 0.18 + 0.18 = 0.36 | 62.4 | 0.18 (A) + 3.2 (B) | 71.4 | 0.53 | 7.98 |
| 0.27 + 0.27 = 0.54 | 71.9 | 0.27 (A) + 4.9 (B) | 87.3 | 0.83 | 11.80 |

Table 2 shows the APTT of Compound A alone or in different mixtures with Compound B. The two last columns show the concentration of A and B that is required in order to reach the same APTT prolongation as that of the mixture.

When the results from Table 2 were applied to Berenbaum's equation the following fractions were obtained:

|  | A($\mu$mol/L) | B($\mu$mol/L) |  |
|---|---|---|---|
| Mixture 1: | (0.08/0.21) + | (1.5/3.87) | = 0.38 + 0.38 = 0.76 |
| Mixture 2: | (0.18/0.53) + | (3.2/7.98) | = 0.34 + 0.40 = 0.74 |
| Mixture 3: | (0.27/0.83) + | (4.9/11.8) | = 0.33 + 0.41 = 0.74 |

Thus, the synergy criterion, according to Berenbaum, was fulfilled. A true synergistic effect was exhibited.

**Claims**

1. A combination product comprising:

   (A) melagatran or a salt, solvate, or prodrug thereof, wherein the prodrug is of the formula

      $R^1O_2C\text{-}CH_2\text{-}(R)Cgl\text{-}Aze\text{-}Pab\text{-}OH$,

   wherein $R^1$ represents $C_{1\text{-}10}$ alkyl or benzyl and the OH group replaces one of the amidino hydrogens in Pab and (B) a Factor VIIa inhibitor or a salt or solvate thereof,

   wherein each of components (A) and (B) is formulated in admixture with a pharmaceutically-acceptable adjuvant, diluent or carrier.

2. A combination product as claimed in Claim 1 which comprises a pharmaceutical formulation including melagatran, the salt, the solvate or the prodrug thereof, and the Factor VIIa inhibitor, the salt or the solvate thereof, and a pharmaceutically-acceptable adjuvant, diluent or carrier.

3. A combination product as claimed in Claim 1 which comprises a kit of parts comprising components:

(a) a pharmaceutical formulation including melagatran, the salt, the solvate or the prodrug thereof, in admixture with a pharmaceutically-acceptable adjuvant, diluent or carrier; and
(b) a pharmaceutical formulation including the Factor VIIa inhibitor, the salt or the solvate thereof, in admixture with a pharmaceutically-acceptable adjuvant, diluent or carrier,

which components (a) and (b) are each provided in a form that is suitable for administration in conjunction with the other.

4. A kit of parts as claimed in Claim 3, wherein components (a) and (b) are suitable for sequential, separate and/or simultaneous use in the treatment of a condition where anticoagulant therapy is indicated.

5. A combination product as claimed in any one of Claims 1 to 4, wherein, in the prodrug, $R^1$ represents linear or branched $C_{1-6}$ alkyl.

6. A combination product as claimed in Claim 5, wherein $R^1$ represents methyl, ethyl, *n*-propyl, *i*-propyl or *t*-butyl.

7. A combination product as claimed in Claim 6, wherein $R^1$ represents ethyl.

8. A method of making a kit of parts as defined in any one of Claims 3 to 7, which method comprises bringing a component (a), as defined in any one of Claims 3 to 7, into association with a component (b), as defined in any one of Claims 3 to 7, thus rendering the two components suitable for administration in conjunction with each other.

9. kit of parts comprising:

(I) one of components (a) and (b) as defined in any one of Claims 3 to 7; together with
(II) instructions to use that component in conjunction with the other of the two components.

10. The use of a combination product as defined in any one of Claims 1 to 7 or a kit of parts as defined in Claim 9 for the manufacture of a medicament for the treatment or prophylaxis of a condition where anticoagulant therapy is indicated.

11. The use of melagatran or a salt, solvate, or prodrug thereof, wherein the prodrug is of the formula

$$R^1O_2C\text{-}CH_2\text{-}(R)Cgl\text{-}Aze\text{-}Pab\text{-}OH,$$

wherein $R^1$ represents $C_{1-10}$ alkyl or benzyl and the OH group replaces one of the amidino hydrogens in Pab and a Factor VIIa inhibitor or a salt or solvate thereof for the manufacture of a medicament for the treatment or prophylaxis of a condition where anticoagulant therapy is indicated.

**Patentansprüche**

1. Kombinationsprodukt, umfassend:

(A) Melagatran oder ein Salz, Solvat oder Prodrug davon, wobei das Prodrug die Formel $R^1O_2C\text{-}CH_2\text{-}(R)Cgl\text{-}Aze\text{-}Pab\text{-}OH$ hat, und wobei $R^1$ ein $C_{1-10}$-Alkyl oder Benzyl darstellt und die OH-Gruppe eines der Amidino-Wasserstoffatome in Pab ersetzt; und
(B) einen Faktor VIIa-Inhibitor oder ein Salz oder Solvat davon,

wobei jede der Komponenten (A) und (B) im Gemisch mit einem pharmazeutisch annehmbaren Hilfsmittel, Verdünnungsmittel oder Träger formuliert ist.

2. Kombinationsprodukt nach Anspruch 1, umfassend eine pharmazeutische Formulierung, welche Melagatran, das Salz, Solvat oder Prodrug davon enthält, und den Faktor VIIa-Inhibitor, das Salz oder Solvat davon, und ein pharmazeutisch annehmbares Hilfsmittel, Verdünnungsmittel oder einen pharmazeutisch annehmbaren Träger.

3. Kombinationsprodukt nach Anspruch 1, umfassend ein Kit aus Teilen, umfassend die Komponenten:

(a) eine pharmazeutische Formulierung, die Melagatran, das Salz, Solvat oder Prodrug davon im Gemisch mit einem pharmazeutisch annehmbaren Hilfsmittel, Verdünnungsmittel oder Träger enthält; und
(b) eine pharmazeutische Formulierung, die den Faktor VIIa-Inhibitor, das Salz oder Solvat davon im Gemisch mit einem pharmazeutisch annehmbaren Hilfsmittel, Verdünnungsmittel oder Träger enthält,

wobei die Komponenten (a) und (b) jeweils in einer Form bereitgestellt werden, die sich zur gemeinsamen Verabreichung mit der anderen eignet.

4. Kit aus Teilen nach Anspruch 3, wobei sich die Komponenten (a) und (b) für eine aufeinanderfolgende, gesonderte und/oder gleichzeitige Verwendung bei der Behandlung eines Zustands eignen, bei dem eine Antikoagulanztherapie angezeigt ist.

5. Kombinationsprodukt nach einem der Ansprüche 1 bis 4, wobei in dem Prodrug $R^1$ ein lineares oder verzweigtes $C_{1-6}$-Alkyl darstellt.

6. Kombinationsprodukt nach Anspruch 5, wobei $R^1$ Methyl, Ethyl, n-Propyl, i-Propyl oder t-Butyl darstellt.

7. Kombinationsprodukt nach Anspruch 6, wobei $R^1$ Ethyl darstellt.

8. Verfahren zur Herstellung eines Teile-Kits nach einem der Ansprüche 3 bis 7, wobei das Verfahren das Zusammenbringen einer Komponente (a) nach einem der Ansprüche 3 bis 7 mit einer Komponente (b) nach einem der Ansprüche 3 bis 7 umfasst, wodurch die beiden Komponenten zur gemeinsamen Verabreichung geeignet gemacht werden.

9. Teile-Kit, umfassend:

(I) eine der Komponenten (a) und (b) nach einem der Ansprüche 3 bis 7; zusammen mit
(II) Anweisungen zur Verwendung dieser Komponente zusammen mit der anderen der beiden Komponenten.

10. Verwendung eines Kombinationsproduktes nach einem der Ansprüche 1 bis 7 oder eines Teile-Kits nach Anspruch 9 zur Herstellung eines Medikamentes für die Behandlung oder Prophylaxe eines Zustands, bei dem eine Antikoagulanztherapie angezeigt ist.

11. Verwendung von Melagatran oder eines Salzes, Solvates oder Prodrugs davon, wobei das Prodrug die Formel $R^1O_2C\text{-}CH_2\text{-}(R)$Cgl-Aze-Pab-OH hat, und wobei $R^1$ ein $C_{1-10}$-Alkyl oder Benzyl darstellt und die OH-Gruppe eines der Amidino-Wasserstoffatome in Pab ersetzt; und eines Faktor VIIa-Inhibitors oder eines Salzes oder Solvates davon, zur Herstellung eines Medikamentes für die Behandlung oder Prophylaxe eines Zustands, bei dem eine Antikoagulanztherapie angezeigt ist.

**Revendications**

1. Produit combiné comprenant :

(A) du mélagatran ou un sel, un solvate ou un précurseur de médicament de celui-ci, le précurseur de médicament ayant la formule

$$R^1O_2C\text{-}CH_2\text{-}(R)Cgl\text{-}Aze\text{-}Pab\text{-}OH$$

dans laquelle $R^1$ représente un groupe alkyle en $C_1$ à $C_{10}$ ou benzyle et le groupe OH remplace un des hydrogènes amidino dans Pab et
(B) un inhibiteur du facteur VIIa ou un sel ou un solvate de celui-ci,
dans lequel chacun des composants (A) et (B) est formulé en mélange avec un adjuvant, un diluant ou un support pharmaceutiquement acceptable.

2. Produit combiné selon la revendication 1, qui comprend une formulation pharmaceutique comprenant du mélagatran, le sel, le solvant ou le précurseur de médicament de celui-ci et l'inhibiteur du facteur VIIa, le sel ou le solvate de celui-ci et un adjuvant, un diluant ou un support pharmaceutiquement acceptable.

3. Produit combiné selon la revendication 1, qui comprend un kit de parties, comprenant les composants

   (a) une formulation pharmaceutique comprenant du mélagatran, le sel, le solvate ou le précurseur de médicament de celui-ci, en mélange avec un adjuvant, un diluant ou un support pharmaceutiquement acceptable et
   (b) une formulation pharmaceutique comprenant l'inhibiteur du facteur VIIa, le sel ou le solvate de celui-ci, en mélange avec un adjuvant, un diluant ou un support pharmaceutiquement acceptable

   les composants (a) et (b) étant chacun proposés dans une forme qui est appropriée pour une administration combinée avec l'autre.

4. Kit de parties selon la revendication 3, dans lequel les composants (a) et (b) conviennent pour une utilisation séquentielle, séparée et/ou simultanée dans le traitement d'un état où une thérapie par un anticoagulant est indiquée.

5. Produit combiné selon l'une quelconque des revendications 1 à 4, dans lequel, dans le précurseur de médicament, $R^1$ représente un groupe alkyle en $C_1$ à $C_6$ linéaire ou ramifié.

6. Produit combiné selon la revendication 5, dans lequel $R^1$ représente un groupe méthyle, éthyle, n-propyle, i-propyle ou t-butyle.

7. Produit combiné selon la revendication 6, dans lequel $R^1$ représente un groupe éthyle.

8. Procédé pour préparer un kit de parties selon l'une quelconque des revendications 3 à 7, ledit procédé comprenant la combinaison d'un composant (a) selon l'une quelconque des revendications 3 à 7 avec un composant (b) selon l'une quelconque des revendications 3 à 7, rendant ainsi les deux composants appropriés pour l'administration combinée les uns avec les autres.

9. Kit de parties comprenant

   (I) un des composants (a) et (b) selon l'une quelconque des revendications 3 à 7 avec
   (II) des instructions pour utiliser ce composant en combinaison avec l'autre des deux composants.

10. Utilisation d'un produit combiné selon l'une quelconque des revendications 1 à 7 ou d'un kit de parties selon la revendication 9 pour la préparation d'un médicament destiné au traitement ou à la prophylaxie d'un état dans lequel une thérapie par un anticoagulant est indiquée.

11. Utilisation de mélagatran ou d'un sel, d'un solvate ou d'un précurseur de médicament de celui-ci, le précurseur de médicament ayant la formule

   $$R^1O_2C\text{-}CH_2\text{-}(R)Cgl\text{-}Aze\text{-}Pab\text{-}OH$$

   dans laquelle $R^1$ représente un groupe alkyle en $C_1$ à $C_{10}$ ou benzyle et le groupe OH remplace un des hydrogènes amidino dans Pab et d'un inhibiteur du facteur VIIa ou d'un sel ou d'un solvate de celui-ci pour la préparation d'un médicament pour le traitement ou la prophylaxie d'un état où une thérapie avec un anticoagulant est indiquée.